(19) Europäisches Patentamt
European Patent Office
Office européen des brevets

(11) Publication number: **0 201 307**
**B1**

(12) # EUROPEAN PATENT SPECIFICATION

(45) Date of publication of patent specification: **28.12.88**

(51) Int. Cl.⁴: **C 07 C 103/133,**
C 07 C 102/08, C 07 C 69/54,
C 07 C 67/20

(21) Application number: **86303399.9**

(22) Date of filing: **06.05.86**

(54) **Process for preparing unsaturated organic compounds.**

(30) Priority: **06.05.85 US 730533**
**29.11.85 US 802879**

(43) Date of publication of application:
**12.11.86 Bulletin 86/46**

(45) Publication of the grant of the patent:
**28.12.88 Bulletin 88/52**

(84) Designated Contracting States:
**DE FR GB IT**

(56) References cited:
**DE-A-1 468 939**
**DE-A-1 618 721**
**GB-A-1 126 054**
**GB-A-1 184 746**

(73) Proprietor: **Rohm and Haas Company**
**Independence Mall West**
**Philadelphia, Pennsylvania 19105 (US)**

(72) Inventor: **Nemec, Joseph William**
**931 Washington Lane**
**Rydal Pa. 19046 (US)**
Inventor: **Smith, Michael James**
**25 Cobblestone Drive**
**Paoli Pa. 19301 (US)**
Inventor: **Wilczynski, Robert**
**1567 Brock Creek Road**
**Yardley Pa. 19067 (US)**

(74) Representative: **Angell, David Whilton et al**
**ROHM AND HAAS (UK) LTD. European**
**Operations Patent Department Lennig House 2**
**Mason's Avenue**
**Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 201 307 B1

**Description**

This invention is concerned with the production of unsaturated organic compounds, for example *alpha, beta*-ethylenically unsaturated amides, acids and esters and especially methacrylamide.

Unsaturated organic compounds are commonly produced by treating a ketone cyanohydrin with concentrated sulfuric acid at an elevated temperature to form an unsaturated amide. The amide may be isolated or reacted with water to form the corresponding unsaturated acid, or reacted with water and alcohol to form the corresponding unsaturated ester. Usually, the reaction product mixture containing the unsaturated amide is reacted with an alcohol, or water and an alcohol, to produce the unsaturated acid and ester, respectively. These processes are described in the technical literature, such as U.S. Patents Nos. 2,101,821; 2,101,822 and 2,140,469.

With the increased emphasis in the chemical process industries on conservation and process economy, attention has been focused on improving yields and reducing energy requirements.

Methacrylamide ("MAM") can be produced by heating a mixture of high strength sulfuric acid and acetone cyanohydrin ("ACN"). MAM is produced from the intermediate *alpha*-sulphato isobutyramide ("SIBAM"). The presence of adventitious water in the system results in some of the desired intermediate SIBAM being converted to undesired *alpha*-hydroxyisobutyramide ("HIBAM"). The HIBAM remains as by-product unless the reaction temperature is raised to about 150°C, whereupon the HIBAM also converts, at least in part, to MAM. However, the reaction rate of this conversion is low even at the high temperature. Also, operation at the high temperature results in the formation of undesired by-products.

GB—A—1,184,746 discloses the production of MAM by heating a mixture of ACN and concentrated sulfuric acid, in which the ratio concentrated sulfuric acid: ACN is at least 1.5:1.

Water promotes the formation of HIBAM and before this invention it was not considered possible to totally exclude water or to conveniently remove water as it is formed. The presence of water promotes by-product HIBAM, which in turn reduces the yield of the desired MAM. The higher temperatures and longer reaction times required to convert the unwanted HIBAM to MAM not only waste energy, but also lead to decomposition and hence lower selectivity. Some reduction of the water content of the reaction mixture has been achieved by employing high strength sulfuric acid or conventional dehydrating agents, but yields of unsaturated products still remain below the desired levels, due to low selectivity.

In another route to unsaturated organic compounds, USSR Invention Certificate No. 333,165 published March 21, 1972, teaches the production of methacrylic acid esters by heating a mixture of methacrylonitrile and a minor amount of acetone cyanohydrin with relatively dilute (84—98%) sulfuric acid at 20°C—150°C. The amount of sulfuric acid is not disclosed, but was probably in large excess since the initial reaction temperature is quite low. The mole ratio of methacrylonitrile to acetone cyanohydrin is very high, of the order of 2:1. Accordingly, the major reaction in the process is the hydration of methacrylonitrile to methacrylamide with dilute sulfuric acid. At the same time most of the acetone cyanohydrin is also converted to methacrylamide. In this prior process, water is a necessary reactant for the formation of the amide, and is intentionally added in the form of dilute sulfuric acid. The mole ratio of water to nitrile is at least 1:1.

Applicants have now found that increased yields of unsaturated organic compounds can be obtained from the reaction of high strength sulfuric acid, not intentionally diluted with water, with a cyanohydrin, by contacting the reaction mass with an organic nitrile in a mole ratio of cyanohydrin to nitrile of from 2:1 to 100:1, preferably 10:1 to 50:1. The amount of nitrile used is related to the amount of water adventitiously present in the system, or to the amount of saturated hydroxyamide, e.g. HIBAM, present. An added advantage is that the reaction temperature may be substantially reduced since the production of saturated hydroxyamide, e.g. HIBAM, can be avoided or any such saturated hydroxyamide produced can be converted into unsaturated organic compound, thereby avoiding the problem of decomposition encountered at higher temperatures while at the same time conserving energy and reducing cost.

The present invention enables the yields of unsaturated products to be increased in the reaction of ketone cyanohydrins with sulfuric acid, by reducing the amount of saturated hydroxyamide by-product of the reaction.

According to the present invention there is provided a process for obtaining improved yields of organic compounds, for example alpha, beta-unsaturated amides such as MAM, which comprises reacting sulfuric acid of over 96% strength, preferably about at least 99% strength, with cyanohydrin, in a mole ratio of at least 1:1, and contacting the reaction mass with organic nitrile, the mole ratio of cyanohydrin initially present: nitrile being from 2:1 to 100:1, preferably 10:1 to 50:1.

The present invention will hereinafter often be described with reference to the production of methacrylamide (MAM) from acetone cyanohydrin (ACN) which is a preferred embodiment of the invention.

The following is a reaction sequence for the production of methacrylamide (MAM) from acetone cyanohydrin (ACN):—

(1) 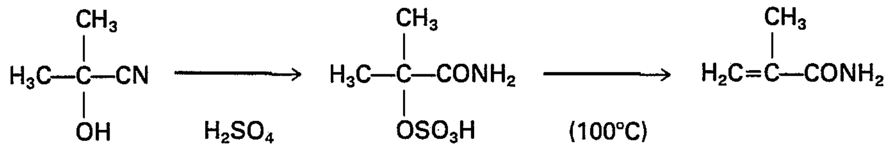


ACN SIBAM MAM

(2) 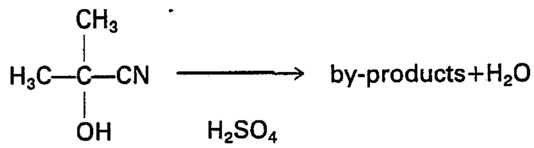


ACN

(3) 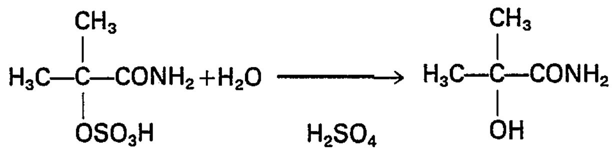


SIBAM HIBAM

(4) 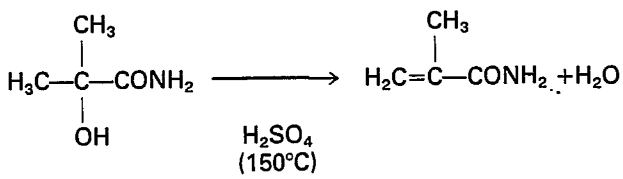


HIBAM MAM

(5) 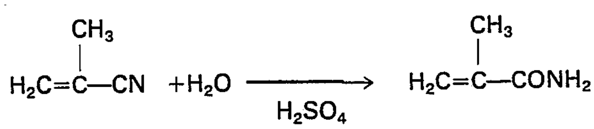


MAN MAM

(6) 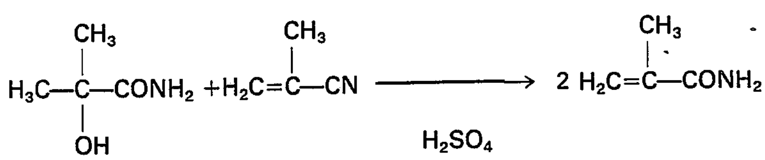


HIBAM MAN MAM

When ACN is reacted with excess sulfuric acid, a mixture of alpha-sulfato isobutyramide (SIBAM), alpha-hydroxyisobutyramide (HIBAM) and by-products is formed, the composition of the mixture being dependent on the amount of water present. As shown in reactions (1) and (3) above, SIBAM or HIBAM is favored depending on the relative amounts of sulfuric acid and water in the reaction mixture. Since SIBAM can be converted to MAM at a lower temperature (about 80°C) and more selectively than can HIBAM (about 150°C), it is evident that the amount of water in the reaction mixture should be reduced as much as possible. In conventional processes this is done by using highly concentrated sulfuric acid, of the order of greater than 98% strength, preferably 99% or higher. Sulfuric acid of 99% strength or higher in this specification is the preferred acid strength. Fuming sulfuric acid is not desirable because it leads to decomposition and thereby reduces yield of useful intermediates and products.

Applicants have now found, in accordance with the present invention, that organic nitriles such as methacrylonitrile (MAN) dehydrate the reaction mixture to form desired MAM, as shown in reaction (5)

above, and surprisingly that the nitrile converts HIBAM to MAM at a much faster rate and more selectively than can be achieved by heating HIBAM in sulfuric acid.

MAN is the preferred nitrile in the production of MAM from ACN because the nitrile itself is converted to the desired MAM. No separation or other purification operation is required when this nitrile is employed.

In contrast to the large excess of nitrile taught in USSR Invention Certificate No. 333,165, the benefits of the present invention are achieved by using excess cyanohydrin and a minor amount of the nitrile. Generally, the cyanohydrin to nitrile mole ratio, may be as low as 2:1, preferably it should be at least 5:1 and may reach as high as 100:1. A more preferred mole ratio is from 10:1 to 50:1 for the conversion of ACN to MAM using MAN, and wherein the amount of MAN is sufficient to react with all the water and HIBAM present. Other ratios outside this 10:1 to 50:1 range, but in the broad range of 2:1 to 100:1, may be used depending on reactants and reaction conditions, including temperature and stage of addition of nitrile, and particularly the amount of water in the system.

The conversion of ACN to MAM takes place at from 30°C to 160°C, preferably from 85° to 130°C. In fact, the conversion of HIBAM to MAM is higher and faster at lower temperature (about 100°C) when MAN is present than at higher temperature (about 150°C) when MAN is absent. Temperatures for conversion of ACN to MAM in the presence of MAN or other nitrile, and similar reactions of the invention, generally will be selected on the basis of good fluidity of the reaction mixture and decomposition temperature, it being desirable that the reaction mixture not be unduly viscous.

Reaction temperature will also depend on the stage at which the nitrile is added. If the nitrile is added prior to admixture of cyanohydrin with sulfuric acid, for example if the nitrile is added with the cyanohydrin, the overall reaction temperature will typically be from 70° to 160°C, preferably from 85° to 130°C. If the nitrile is added after contact between the sulfuric acid and cyanohydrin (delayed addition), the reaction temperature during the additions will be at the lower end of the range 80° to 130°C, e.g. 80° to 120°C, preferably 85° to 120°C) and thereafter at the higher temperature.

The reaction of cyanohydrin, sulfuric acid and nitrile is conveniently conducted with efficient agitation. Preferably, for the production of MAM, the reaction is run in a first stage at a temperature of from 80° to 130°C followed, if required, by a second stage at a temperature of 90°C to 120°C, to promote conversion of SIBAM to MAM. The second stage may be conducted until conversion of intermediates to MAM is substantially complete. An aliphatic alcohol, e.g. methyl alcohol, may be added to the reaction mass during or following the second stage reaction whereby an aliphatic ester of methacrylic acid, e.g. methyl methacrylate, is formed as the principal reaction product. Reaction times will vary according to the reaction system and generally will range from several minutes to several hours for a single stage or multi-stage batch reaction.

The reaction may be run continuously or semi-continuously with adjustment of contact times and throughput rates in accordance with principles well understood by those skilled in the art. While the foregoing concerns atmospheric pressure reactions, the reaction may also be run at subatmospheric and superatmospheric pressures.

The products can be worked up by known techniques such as described in the patents cited above and other technical literature. If desired, polymerization inhibitors, such as a hydroquinone, may be added to the reaction mixture to prevent polymerization.

Other cyanohydrins convertible to useful unsaturated products in accordance with the invention include those derived from symmetrical alkyl ketones such as diethyl, dipropyl, dibutyl or diamyl ketones and unsymmetrical ketones such as methyl ethyl, methyl propyl, methyl isopropyl, methyl butyl or methyl nonyl, also aralkyl aryl or ketones such as benzyl ethyl, tolyl ethyl, dibenzyl and the like.

Organic nitriles in addition to MAN, useful in the invention, include hydrocyanic acid and saturated and unsaturated aliphatic nitriles such as acrylonitrile, acetonitrile and others, singly or in admixture.

In summary, Applicants have found that the use, in accordance with the invention, of organic nitrile, together with reduced water content, can prevent the formation of undesirable intermediates such as HIBAM, or can convert such undesirable intermediates into unsaturated organic compounds. These intermediates are undesirable because they are difficult to convert to useful unsaturated products without undue energy input and processing. The nitriles not only minimize these problems but also, because they can be used in small amounts and permit lower reaction temperatures, lead to better reaction control, higher useful product yields and overall better process economics.

The present invention will now be further illustrated by way of the following Examples.

All parts and percentages are by weight and all temperatures are °C.

Example 1

Illustrates delayed addition of nitrile to increase yield of MAM

To a 500 ml three-neck round bottom flask fitted with a mechanical stirrer was charged sulfuric acid (99.6%, 59.0 g). The flask and contents were heated to 92°C. Using a syringe pump, acetone cyanohydrin (ACN, 39.4 g, 0.463 moles) was added with rapid stirring over a 54 minute period. The contents of the flask were maintained at 94°C to 95°C during the addition period. At the end of the addition period the contents of the flask were cooled to 80°C. Analysis of the reaction mixture showed that it contained 0.302 moles of methacrylamide (MAM), 0.0891 moles of alpha-sulfato isobutyramide (SIBAM), and 0.0861 moles of alpha-hydroxy isobutyramide (HIBAM). Methacrylonitrile (MAN, 4.30 g, 0.0642 moles) was added to the

reaction mixture which was then heated to 120°C and held there for 24 minutes. Analysis indicated 0.447 moles of MAM (84.8%, based on ACN and MAN) and 0.0334 moles of HIBAM.

Example 2
Illustrates addition of nitrile *before* contact of ACN and sulfuric acid.

To a 500 ml three-neck round bottom flask fitted with a mechanical stirrer was charged sulfuric acid (99.6%, 60.9 g). This flask and contents were heated to 92°C. Using a syringe pump, a solution of acetone cyanohydrin (34.0 g, 0.400 moles) and methacrylonitrile (MAN, 3.2 g, 0.0477 moles) were added with rapid stirring over a 54 minute period. The contents of the flask were maintained at 94°C to 95°C during the addition period. At the end of the addition, the flask and contents were heated to 105°C and held for 10 minutes, then taken to 120°C and held for 10 minutes. Analysis of the resulting material indicated organic products as follows: MAM, 0.355 moles, 79.3% based on ACN and MAN; methacrylic acid (MAA), 0.0087 moles, 1.9%; SIBAM, 0.0096 moles, 2.1%; and HIBAM, 0.0412 moles, 9.2%.

Comparative Example 3
The procedure of Example 2 was repeated twice in all essential respects but with 61.09 g sulfuric acid, 39.31 g (0.461 moles) of ACN and no nitrile. Analysis of the reaction mix at the end of the heating period indicated the following as averages of the two runs: MAM and MAA, 0.347 moles, 75.2%; SIBAM, 0.0159 moles, 3.45%; and HIBAM, 0.0846 moles, 18.4%.

Example 4
The procedure of Example 2 was repeated in all essential respects but with 61.05 g of sulfuric acid, 2.76 g (0.052 moles) of acrylonitrile (in place of the MAN), and 36.85 g (0.433 moles) of ACN. Analysis of the reaction mix at the end of the heating period indicated: MAM and methacrylic acid (MAA), 0.360 moles, 83.2%; SIBAM, 0.0087 moles, 2%; and HIBAM, 0.0359 moles, 8.3%. In comparison to Example 3 the yield of HIBAM by-product was reduced from 18.4% to 8.3% and the yield of useful products increased from 75.2% to 83.2%.

To provide further insight into the effects of nitrile on by-products formed in the reactions of Examples 1—4, the reaction of 2-hydroxyisobutyramide (HIBAM) with methacrylonitrile (MAN) in sulfuric acid was studied, as follows.

To a reaction vessel was charged 0.210 grams (2.04 mmoles) of HIBAM, 0.997 grams (10.2 mmoles) of 99.8% sulfuric acid. This mixture was stirred until the HIBAM dissolved. Then to this mixture was added, while stirring, 0.142 grams (2.12 mmoles) of methacrylonitrile (MAN). After stirring at room temperature for 2 hours, analysis indicated that a major portion (>75 mole %) of the MAN/HIBAM mixture converted to a mixture of methacrylamide (MAM) and 2-sulfatoisobutyramide (SIBAM) with some (<25 mole %) MAN and HIBAM remaining. Upon further standing at room temperature, more MAM and SIBAM formed at the expense of MAN and HIBAM. Accountabilities on MAN and HIBAM at this point were near 100 percent.

The product solution was then heated at 100°C for 30 minutes. Analysis indicated that the new mixture yielded 80 mole % MAM with 5 mole % SIBAM and 6 mole % HIBAM remaining (based on total moles originally charged for MAN and HIBAM). The analysis also showed that approximately 9 mole % of various unwanted side products (compounds that do not convert to MAM upon further heating) and formed. Based on other experiments it is believed that half of this decomposition comes from MAN and half from HIBAM and that the SIBAM and HIBAM are produced from original HIBAM. Hence, the yields from HIBAM alone are 70 mole % MAM, 10 mole % SIBAM and 12 mole % HIBAM. Under identical conditions but without any MAN present in the original reaction solution, HIBAM yields only 46 mole % MAM, with 5 mole % SIBAM and 38 mole % HIBAM remaining, along with 11 mole % of decomposition products. The nitrile therefore causes increased yields of desired MAM by reaction with undesired HIBAM.

Similar studies were made on the reaction of HIBAM with acrylonitrile (AN) in sulfuric acid.

To a reaction vessel was charged 0.215 grams (2.09 mmoles) of HIBAM, 0.990 grams (10.1 mmoles) of 99.8% $H_2SO_4$. This mixture was stirred until the HIBAM dissolved. Then to this mixture was added 0.110 grams (2.075 mmoles) of acrylonitrile (AN). After several hours at room temperature analysis indicated that a major portion (ca. 90 mole %) of the HIBAM converted to SIBAM with some (ca. 10 mole %) HIBAM remaining, and that approximately 83 mole % of the AN converted to acrylamide with significant amount (ca. 20 mole %) of decomposition products (i.e., compounds which do not convert to either MAM or acrylamide upon further heating) being formed from the AN. Upon heating for one hour at 100°C, analysis indicated that yields from HIBAM were 91 mole % MAM with 1 mole % SIBAM and 4 mole % HIBAM remaining (only 4 mole % was lost to decomposition products). In addition, there was now an approximately 70 mole % yield of acrylamide from acrylonitrile. Under identical conditions but without AN present in the original reaction solution, the yields from HIBAM were 61 mole % MAM, 3 mole % SIBAM and 25 mole % HIBAM with ca. 11 mole % decomposition products. Accordingly, the nitrile significantly increases the yields of desirable MAM at the expense of undesired HIBAM.

**Claims**

1. A process for the production of unsaturated organic compounds wherein sulfuric acid of over 96%

strength and a cyanohydrin are reacted in a mole ratio of at least 1:1, characterized in that the reaction mass is contacted with an organic nitrile to convert undesired by-product into desired unsaturated organic compound, the mole ratio of cyanohydrin initially present: nitrile being from 2:1 to 100:1.

2. A process as claimed in claim 1 wherein the nitrile is fed to the reaction mass with the cyanohydrin.

3. A process as claimed in claim 1 wherein the nitrile is added to the reaction mass after contact of the sulfuric acid with the cyanohydrin.

4. A process as claimed in claim 1 wherein the sulfuric acid strength is at least 99%, the cyanohydrin is acetone cyanohydrin, and the nitrile is methacrylonitrile.

5. A process as claimed in claim 4 wherein the mole ratio of acetone cyanohydrin to methacrylonitrile is 10:1 to 50:1, the methacrylonitrile is fed to the reaction mass with the acetone cyanohydrin, and the reaction is conducted at a temperature of from 70° to 160°C.

6. A process as claimed in claim 5 wherein the temperature is from 85° to 130°C.

7. A process as claimed in claim 5 wherein the reaction is conducted in a first stage at a temperature of from 80° to 130°C and in a second stage at a temperature of from 90° to 120°C, until conversion of intermediates to methacrylamide is substantially complete.

8. A process as claimed in claim 4 wherein the mole ratio of acetone cyanohydrin to methacrylonitrile is 10:1 to 50:1, the methacrylonitrile is added to the reaction mass after contact between the sulfuric acid and acetone cyanohydrin, and the reaction is conducted at a temperature of from 80° to 130°C.

9. A process as claimed in claim 8 wherein the temperature is from 85° to 120°C.

10. A process as claimed in claim 8 wherein the reaction is conducted in a first stage at a temperature of from 80° to 130°C and in a second stage at a temperature which is lower than that of the first stage and is from 90° to 120°C.

11. A process as claimed in claim 7 or claim 10 wherein an aliphatic alcohol is added to the reaction mass during or following the second stage reaction, whereby an aliphatic ester of methacrylic acid is formed as the principal reaction product.

12. A process as claimed in claim 11 wherein the aliphatic alcohol is methyl alcohol, and the principal reaction product is methyl methacrylate.

**Patentansprüche**

1. Verfahren zur Herstellung von ungesättigten organischen Verbindungen, bei welchem Schwefelsäure mit einer Stärke von mehr als 96% und ein Cyanohydrin in einem Molverhältnis von wenigstens 1:1 umgesetzt werden, dadurch gekennzeichnet, daß die Reaktionsmasse mit einem organischen Nitril zur Umwandlung von unerwünschtem Nebenprodukt zu einer gewünschten ungesättigten organischen Verbindung kontaktiert wird, wobei das Molverhältnis von Cyanohydrin, das anfänglich vorhanden ist, zu dem Nitril 2:1 bis 100:1 beträgt.

2. Verfahren nach Anspruch 1, worin das Nitril der Reaktionsmasse mit dem Cyanohydrin zugeführt wird.

3. Verfahren nach Anspruch 1, worin das Nitril der Reaktionsmasse nach einem Kontakt der Schwefelsäure mit dem Cyanohydrin zugesetzt wird.

4. Verfahren nach Anspruch 1, worin die Stärke der Schwefelsäure wenigstens 99% beträgt, das Cyanohydrin aus Acetoncyanohydrin besteht und das Nitril Methacrylnitril ist.

5. Verfahren nach Anspruch 4, worin das Molverhältnis von Acetoncyanohydrin zu Methacrylnitril 10:1 bis 50:1 beträgt, das Methacrylnitril der Reaktionsmasse mit dem Acetoncyanohydrin zugeführt wird und die Reaktion bei einer Temperatur von 70°C bis 160°C durchgeführt wird.

6. Verfahren nach Anspruch 5, worin die Temperatur 85 bis 130°C beträgt.

7. Verfahren nach Anspruch 5, worin die Reaktion in einer ersten Stufe bei einer Temperatur von 80 bis 130°C und in einer zweiten Stufe bei einer Temperatur von 90 bis 120°C durchgeführt wird, bis die Umwandlung von Zwischenprodukten zu Methacrylamid im wesentlichen beendet ist.

8. Verfahren nach Anspruch 4, worin das Molverhältnis von Acetoncyanohydrin zu Methacrylnitril 10:1 bis 15:1 beträgt, das Methacrylnitril der Reaktionsmasse nach dem Kontakt zwischen der Schwefelsäure und dem Cyanohydrin zugesetzt wird und die Reaktion bei einer Temperatur von 80 bis 130°C durchgeführt wird.

9. Verfahren nach Anspruch 8, worin die Temperatur 85 bis 120°C beträgt.

10. Verfahren nach Anspruch 8, worin die Reaktion in einer ersten Stufe bei einer Temperatur von 80 bis 130°C und in einer zweiten Stufe bei einer Temperatur durchgeführt wird, die niedriger ist als diejenige der ersten Stufe und zwischen 90 und 120°C liegt.

11. Verfahren nach Anspruch 7 oder Anspruch 10, worin ein aliphatischer Alkohol der Reaktionsmasse während oder anschließend an die Reaktion der zweiten Stufe zugesetzt wird, wobei ein aliphatischer Ester von Methacrylsäure als Hauptreaktionsprodukt gebildet wird.

12. Verfahren nach Anspruch 11, worin der aliphatische Alkohol Methylalkohol ist und das Hauptreaktionsprodukt Methylmethacrylat ist.

**Revendications**

1. Un procédé pour la production de composés organiques insaturés dans lequel on fait réagir de

l'acide sulfurique ayant une concentration supérieure à 96% et une cyanhydrine dans un rapport molaire d'au moins 1/1, caractérisé en ce que la masse réactionnelle est mise au contact d'un nitrile organique pour transformer un sous-produit indésirable en un composé organique insaturé désiré, le rapport molaire de la cyanhydrine initialement présente au nitrile étant de 2/1 à 100/1.

2. Un procédé selon la revendication 1, dans lequel le nitrile est introduit dans la masse réactionnelle avec la cyanhydrine.

3. Un procédé selon la revendication 1, dans lequel le nitrile est ajouté à la masse réactionnelle après contact de l'acide sulfurique avec la cyanhydrine.

4. Un procédé selon la revendication 1, dans lequel la concentration de l'acide sulfurique est d'au moins 99%, la cyanhydrine est l'acétonecyanhydrine et le nitrile est le méthacrylonitrile.

5. Un procédé selon la revendication 4, dans lequel le rapport molaire de l'acétonecyanhydrine au méthacrylonitrile est de 10/1 à 50/1, le méthacrylonitrile est introduit dans la masse réactionnelle avec l'acétonecyanhydrine et la réaction est effectuée à une température de 70° à 160°C.

6. Un procédé selon la revendication 5, dans lequel la température est de 85° à 130°C.

7. Un procédé selon la revendication 5, dans lequel la réaction est effectuée dans un premier stade à une température de 80° à 130°C et dans un second stade à une température de 90° à 120°C jusqu'à ce que la conversion des intermédiaires en méthacrylamide soit essentiellement complété.

8. Un procédé selon la revendication 4, dans lequel le rapport molaire de l'acétonecyanhydrine au méthacrylonitrile est de 10/1 à 50/1, le méthacrylonitrile est ajouté à la masse réactionnelle après contact entre l'acide sulfurique et l'acétonecyanhydrine et la réaction est effectuée à une température de 80° à 130°C.

9. Un procédé selon la revendication 8, dans lequel la température est de 85° à 120°C.

10. Un procédé selon la revendication 8, dans lequel la réaction est effectuée dans un premier stade à une température de 80° à 130°C et dans un second stade à une température qui est inférieure à celle du premier stade et qui est comprise de 90° à 120°C.

11. Un procédé selon la revendication 7 ou la revendication 10, dans lequel on ajoute un alcool aliphatique à la masse réactionnelle pendant ou après le second stade de réaction pour former un ester aliphatique de l'acide méthacrylique comme produit réactionnel principal.

12. Un procédé selon la revendication 1, dans lequel l'alcool aliphatique est l'alcool méthylique et le produit réactionnel principal est le méthacrylate de méthyle.